# EUROPEAN PATENT APPLICATION

(11) **EP 0 534 014 A1**
(43) Date of publication of application: **31.03.1993**
(21) Application number: 91250259.8
(22) Date of filing: 25.09.1991
(51) Int. Cl.: A61L 33/00, A61L 27/00, A61L 29/00, A61L 31/00

(54) **Non-adhesive biocompatible surface**

(71) Applicant: W.R. Grace & Co.-Conn., New York, New York 10036 (US)
(72) Inventor: Mori, Yuichi, 1642-212-B-4 Kamariva-cho, Kanagawa-ken (JP); Mikaki, Masato, Room No. 305, Kanagawa-ken (JP); Osada, Yoshihito, Mito-shi, Ibaragi-ken (JP)
(74) Representative: UEXKÜLL & STOLBERG

(57) **Abstract**

This invention provides a biocompatible material comprising a temperature responsive polymer whichprevents adhesion of living body elements, particularly blood components.

## Description

### Technical Field

This invention relates to a biocompatible material that prevents adhesion of living body elements. More specifically, this invention relates to a body compatible material, particularly a blood compatible material, that prevents adhesion of living body components, particularly adhesion of blood components. The surface of the material is substantially non-adhesive to living body elements when contacted with fluids comprising living body tissue, particularly blood.

### BACKGROUND OF THE INVENTION

With today's rapid advancement of medicine, medical diagnostic equipment that is used in direct contact with living body element, particularly with blood, have become available to the clinical field. Materials used in therapeutic and diagnostic instruments must have the following dual requirements. The materials must fulfill the medical functionality, such as sensing function, substance-delivery function, selective adsorbability of substances, selective permeability of substances, electrical property, optical property, adhesiveness, durability, and mechanical strength. The materials also must be compatible with living body fluid.

When conventional artificial material is contacted with living body elements, the living body element adheres onto the surface of the material. Particularly when it is contacted with blood, a thrombus often is formed. Thrombus formation significantly hinders the performance of the therapeutic and diagnostic instrument.

Furthermore, adhesion of living body elements, when the therapeutic or diagnostic equipment is used inside the living body, will cause the following serious side reactions: (1) Blood elements deposited on the surface of medical material, particularly thrombus detaches from the surface of the material by blood flow, and this free thrombus will blockade the important blood vessels in various organs and tissue to cause necrosis such as cerebral embolism, pulmonary infarction, and myocardial infarction, (2) living body elements, particularly thrombus, deposited on the surface of medical material will serve as a bed suitable for growth of bacteria and fungi to cause fever and bacteremia, (3) the thrombus formed on the surface of medical material consumes blood elements such as fibrinogen, coagulating factors and platelets, to cause hemorrhagic tendency. And, if a large amount of thrombus is formed in the body, it will also consume the fibrinolytic enzymes that dissolve the thrombus, and therefore it may induce DIC (disseminated intravascular coagulation) wherein both thrombosis and hemorrhagic trend are seen. Particularly, risk of DIC is high with artificial organs, such as artificial cardiopulmonary device, cardiac assist device, and artificial liver where blood contacts with the material in large surface area. Therefore, if deposition of aforesaid living body elements can be prevented without causing a change to the properties of the blood, it can be expected to have a significant contribution to the field of artificial organs and medical diagnostic equipments. From this aspect, active development of medical materials that have biocompatibility has been carried out for some time.

Existing biocompatible materials developed so far can be divided roughly into two groups, namely (1) materials that weakens the interaction between the surface of the material and the living body elements such as blood to suppress adhesion of the living body elements or its damage, and (2) materials that have immobilized the drug that specifically suppresses the thrombus formation or physiologicallY active substance that dissolves the formed thrombus, on the material. Because this invention is related particularly to the former material, past studies conducted on the former materials are explained in detail below.

Materials that weaken the interaction between the material surface and the living body element include: (1) materials that have a soluble surface where at least a hydrophilic polymer exists on the surface of the material, and (2) materials that have a micro phase separation structure having both hydrophilic regions and hydrophobic regions developed to prevent adhesion or attachment of living body elements.

A typical example of the surface soluble materials is the material having cilia made of hydrophilic and soft polymers on the surface of the material. Attachment of living body component onto the surface of the material is suppressed by the osmotic pressure effect of the cilia which are dissolved locally and also by their exclusive volume effect. Examples of materials belonging to this group include materials that have polyethylene-glycol grafted on the surface (Y. Mori, et al.: *Jpn. J. Artif. Organs*, 10, 993, 1981), materials that have polyacrylamide grafted on the surface (K. Hayashi, et al.: *Biomaterials*, 1, 59, 1983), materials that have polyvinyl alcohol grafted on the surface (Y. Ikada, et al.: *J. Biomed. Mater. Res*., 15, 697, 1981).

The detailed mechanism of prevention of attachment of living body elements to the material having the micro phase separation structure is currently unclear. However, *in vivo* systems, such as cell membrane, it has been known that a micro phase separation structure composed of lipid layer having hydrophobicity and proteins having hydrophilicity in mosaic fashion is organized. The micro phase separation structure is considered to prevent the attachment of living body components due to the similarity to the *in vivo* systems. These micro phase separation structures are prepared by various techniques, such as blend, block or graft polymerization, crystalline/noncrystalline formation, addition of filler, porositization and roughening of the surface and so on. Particularly, the structure that has a high living body element attachment prevention effect is said to be a micro phase separation structure made of hydrophilic region and hydrophobic region which are noticed with blend, block or graft polymers. Thus, as the hydrophobic element, the block polymers made of polystyrene, polydimethylsiloxane or polybutadiene and poly-2-hydroxyethyl methacrylate as the hydrophilic element have been developed (M. Okano, et al.: *Jpn. J. Artif. Organs,* 11, 1167, 1982). The ability of said block polymer to attach platelet which is a blood element is said to be significantly suppressed, compared with the respective hydrophobic homopolymers and hydrophilic homopolymers. It is known, in this case, that size of the hydrophilic and hydrophobic regions is an important factor to control the ability to attach the living body elements. Beside, polystyrene/polyamine graft polymers, polystyrene/polybenzyl glutamate graft polymers, polyvinyl alcohol/polyacrylonitrile graft polymers, polydimethyl-siloxane/polyamino acid block and graft polymers have also been developed as biocompatible materials that have micro phase separation structure.

The materials which have been so far developed to prevent attachment of living body element have been reviewed. However, these materials still face many problems from viewpoints of biocompatibility, particularly blood compatibility.

The hydrophilic polymers on the surface of the material may suppress the attachment of blood elements. However, the hydrophilic polymers on the surface are known to cause activation of the blood elements. Particularly, it has been recognized that the polar groups of hydrophilic polymers, compared to hydrophobic polymers, can drastically activate the blood coagulating factors and the complements (N. Ogata: *Handbook of New Polymeric Materials*, page 307, 1989). And the material prepared by grafting a hydrophilic polymer such as polyacrylamide or poly-2-hydroxyethyl methacrylate on the surface has been found to increase the damage on the circulating blood platelets when the grafted amount of hydrophilic polymer was increased, although the amount of thrombus attached on the surface of the material may surely decrease, when the grafted material was left in blood (B. D. Ratner, et al.: *J. Polym. Sci., Polym*. *Symp*., 66, 363, 1979). These evidences suggest that the hydrophilicity of the material may suppress attachment of blood elements but cause damage to blood elements such as blood coagulating factor, complement system, and platelet, while hydrophobicity of the material may enhance attachment of blood elements but can suppress significantly the damage to the blood elements. Therefore, satisfactory biocompatibility could not be achieved with the material that has hydrophilic polymer alone on the surface.

On the other hand, as to the materials having micro phase separation structure made of hydrophilic region and hydrophobic region, the biocompatibility may change significantly with the size and morphology of each region, as previously mentioned (M. Okano, et al.: *Jpn. J. Artif. Organs*, 11, 1167, 1982). In case of blend, block, and graft polymers made of hydrophilic and hydrophobic polymers, size and morphology of the hydrophilic and hydrophobic regions in the micro phase separation structure are affected significantly by the composition ratio of hydrophobic polymer and hydrophilic polymer, chemical affinity between hydrophilic polymer and hydrophobic polymer, and method of its fabrication, and thus it is extremely difficult to form a reproducible micro phase separation structure. Therefore, in case of the materials having micro phase separation structure, it is extremely difficult to acquire a reproducible and satisfactory biocompatibility (or bioadaptability). Furthermore, in case of said materials, hydrophobic property may change with the quick attachment of living body elements on the hydrophobic region to easily lose the clear micro phase separation structure when they are exposed to fluid of living body. Particularly in the environment that allows the living body elements to attach easily, in other words in the occasion when it was transplanted or embedded under the skin where body fluid tends to stagnate or when it is left in the area where the blood flow is slower, it has a serious flaw that causes significant attachment of living body elements or formation of thrombus.

So far, we have reviewed the biocompatible materials of the prior art, but their performance is insufficient. Thus, when applied in various artificial organs or medical diagnostic equipments, significant decline of the performance of the equipment caused by attachment of the living body elements or serious side effects such as bacteremia or DIC caused by tissue necrosis or thrombosis with the free thrombus, as described before, has often been found.

### SUMMARY OF THE INVENTION

The objective of this invention is to provide a material which is substantially nonadhesive to living body elements and a method for using the material. The living body elements are contained in a fluid, particularly blood. The material comprises a temperature responsive polymeric compound which is maintained at a temperature near the LCST of the polymer.

Another object of the invention is to provide a medical material which has a longer life-time. The longer life is due to substantial prevention of adherence of living body element to the material which reduces damage to the medical material.

The present invention provides a surface which is repellent to living body elements that has reduced serious side effect that might be caused by attachment of the living body elements and have enhanced medical functionality. Specifically, the present invention substantially prevents thrombus formation.

### BRIEF DESCRIPTION OF THE DRAWINGS

Fig. 1 illustrates the dependency of the contact angle of the PNIPAAm coated surface against water on temperature.

Fig. 2 illustrates the dependency of the contact angle of various polymer-coated surfaces against water on temperature.

Fig. 3 is scanning electron microphotographs to show attachment of blood platelets on polyester film and PNIPAAm coated film, when each film was contacted with fresh, platelet-rich plasma of dog.

Fig. 4 is scanning electron microphotograph to show attachment of blood platelets on PNIPAAm and Cop.(NIPAAm/AAm)-2 coated films.

### DETAILED DESCRIPTION OF THE INVENTION

This invention provides a new material that substantially repels attachment of living body elements. The material of this invention comprises a temperature-responsive polymeric compound that has its LCST near the temperature of use.

LCST (Lower Critical Solution Temperature) refers to a transition temperature of the temperature-responsive polymeric compound for hydration and dehydration.

Living body element refers to components found in body fluids, particularly those found in blood and plasma. Living body element includes, but is not limited to, plasma proteins, blood proteins, blood cells, membrane components, etc. Living body elements refers particularly to blood coagulating factors, complement factors, blood platelets, etc.

The material of this invention employs a temperature-responsive polymeric compound having its LCST near the use temperature. For the purpose of this application, a temperature near the LCST of a temperature responsive polymer is within 5°C, preferably within 3°C, of the LCST of the polymer. The shape, structure and other constitutive elements of the material are not particularly limited as long as the objective of this invention can be attained.

The material of this invention comprises a temperature-responsive polymeric compound alone or in combination with a supporting material. The material of the invention may have a crosslinked structure incorporated in said polymeric compound. Adherence of the polymeric compound to the supporting member may be accomplished by commonly known means, such as by coating or by graft polymerization. The support is at least partially in adherence with the polymeric compound.

The temperature-responsive polymeric compound to be used in this invention shows hydrophobicity at a temperature higher than the LCST and changes to hydrophilicity at a temperature below the LCST, and such change is characterized by being thermally reversible.

Change of state of the temperature-responsive polymeric compound is said to be caused by hydration and dehydration. This has been explained by Haskins, M., et al. in *J. Macromol. Sci. Chem*., A2 (8), 1441, 1968, using as the sample the poly-N-isopropyl acrylamide (PNIPAAm) which is one of such polymeric compounds. PNIPAAm is a polymeric compound which has a negative temperature coefficient of solubility. PNIPAAm shows hydrophilic property at lower temperature because a hydrate (oxonium hydroxide) which depends on the hydrogen bond of PNIPAAm molecule and water molecule is formed at low temperature. However, because oxonium hydroxide degrades and dehydrates when temperature is raised above the LCST, the PNIPAAm molecule becomes hydrophobic and precipitates as a result.

Aforesaid change can be observed similarly in the system where crosslinks were incorporated in said polymeric compound and also in the system where said polymer was graft-polymerized. Therefore, near LCST, said polymeric compound shifts between two phases, i.e., hydrophobic phase and hydrophilic phase, and therefore is very unstable.

Generally speaking, interaction among hydrophilic substances having polar groups is caused by a bonding force among the dipoles of polar groups. The affinity among hydrophobic substances that do not have polar groups is caused by the hydrophobic bonding force of water molecules existing therebetween. It is believed that such interaction does not exist between hydrophobic substance and hydrophilic substance that do not have such binding force.

On the other hand, living body elements, particularly the plasma proteins and various blood cell membranes are composed of elements having high hydrophilic properties, elements having high hydrophobic properties, or elements having both hydrophilic and hydrophobic properties. Therefore, when these blood elements interact with said polymeric compound, said polymeric compound will show hydrophobicity when the temperature is sufficiently higher than the LCST of said polymeric compound. Therefore, blood elements having high hydrophobicity or the hydrophobic part in the elements and said polymeric compound may interact selectively. In contrast, if the temperature is sufficiently lower than the LCST, said polymeric compound will show a high hydrophilicity and as a result the blood elements having high hydrophilicity or the hydrophilic part in the elements will interact selectively with the polymeric compound. On the other hand, if the temperature is near the LCST or if the temperature changes above and below the LCST, said polymeric compound will be either in the intermediary state of hydrophilicity and hydrophobicity or will shift between the two phases. Under this state, the blood elements cannot interact stably with said polymeric compound, and therefore the blood elements cannot attach to the said polymeric compound near the LCST. Even when they can attach to the polymeric compound, the force of such attachment will be extremely weak.

Poly-N-substituted acrylamide derivatives, poly-N-substituted methacrylamide derivatives and their copolymers, polyvinylmethyl ether, polyethylenoxide, etherized methylcellulose, and partially acetylated polyvinyl alcohol can be mentioned as the temperature-responsive polymeric compounds that can be used as the substrate of this invention. Particularly desirable is poly-N-substituted acrylamide derivatives or poly-N-substituted methacrylamide derivatives or their copolymers, polyvinylmethyl ether, and partially acetylated polyvinyl alcohols.

Preferred polymeric compounds are listed below, in the order of lower LCST.
Poly-N-acryloyl piperidine;
Poly-N-n-propyl methacrylamide;
Poly-N-isopropyl acrylamide;
Poly-N,N-diethyl acrylamide;
Poly-N-isopropyl methacrylamide;
Poly-N-cyclopropyl acrylamide;
Poly-N-acryloyl pyrrolidine;
Poly-N,N-ethylmethyl acrylamide;
Poly-N-cyclopropyl methacrylamide;
Poly-N-ethyl acrylamide
Aforesaid polymers may be homopolymers or may be copolymers with other monomers. Hydrophilic monomers and hydrophobic monomers can be used as the monomers for copolymerization. Generally, copolymerization with hydrophilic monomers raises the LCST and copolymerization with hydrophobic monomers lowers the LCST. Therefore, a polymeric compound having a desired LCST can be obtained by selection of these monomers.

N-Vinylpyrrolidone, vinylpyridine, acrylamide, methacrylamide, N-methyl acrylamide, hydroxyethyl methacrylate, hydroxyethyl acrylate, hydroxymethyl methacrylate, hydroxymethyl acrylate, acrylic acid and methacrylic acid and their salts, vinyl sulfonic acid and styrylsulfonic acid that have acidic group, and N,N-dimethylaminoethyl methacrylate, N,N-diethyl-aminoethyl methacrylate, N,N-dimethylaminopropyl acrylamide and its salts that have basic group can be mentioned as the hydrophilic monomers, but it is not limited to these examples.

On the other hand, acrylate derivatives and methacrylate derivatives such as ethyl acrylate, methyl methacrylate, and glycidyl methacrylate and so on, N-substituted alkyl methacrylamide such as N-n-butyl methacrylamide and so on, vinyl chloride, acrylonitrile, styrene and vinyl acetate can be mentioned as the hydrophobic monomers, but it is not limited to these examples.

The shape of the substrate of this invention is not particularly defined, and it can be film, sheet, tube, particle, fiber, sponge and so on. Any ordinary molding method of polymeric compound can be used to form these shapes. For example, a desirable shape can be formed by a method by which the polymer is dissolved in water or an organic solvent and the solution is formed into a film or a sheet by solvent casting process; a method by which an aqueous polymer solution being cooled below LCST is extruded into water at a temperature above LCST or water-immiscible organic solvent through a nozzle to form a film, particle, tube or fiber; or a method by which the polymer is formed directly into particles by suspension polymerization or precipitation polymerization process.

To prepare a substrate by coating the polymer on the surface of a supporting material, a supporting material having a desired shape is prepared ahead of time and the polymer may be coated on the surface of the supporting material by any of the ordinary methods.

In case of graft-polymerizing the polymeric compound on the surface of a supporting member, various types of monomers and graft copolymerization methods can be used to adjust to a desired LCST as described before, in addition to selecting the polymeric compound having a desired LCST of itself. In case of graft copolymerization method, hydrophilic monomers or hydrophobic monomers can be used. Generally, copolymerization with hydrophilic monomer will raise the LCST of the graft copolymer, and copolymerization with a hydrophobic monomer will depress the LCST. Hydrophilic monomers and hydrophobic monomers that can be used have been described above.

Materials which have been usually used as the medical material are preferred as the supporting material in the case of graft-polymerizing the polymeric compound on the surface. Glass, polystyrenes, polycarbonates, polymethacrylates, polypropylenes, polyethylenes, polyesters, polyamides, polyvinylidenefluorides, polyoxymethylenes, polyvinyl-chlorides, polyacrylnitriles, polytetrafluoroethylenes, polydimethylsiloxanes, cellulosic polymers, and so on can be mentioned as examples, but it is not limited to these examples.

Most suitable method of graft polymerization of the temperature-responsive monomers to the supporting member can be selected, based on the material and the shape. Low temperature plasma polymerization technique can be used favorably when the supporting member is a sheet, film, or flat membrane. With this method, graft polymerization is possible only on the surface of the supporting material, because it can be graft polymerized easily on the polymers such as polypropylenes, polyethylenes, polytetrafluoroethylenes, polydimethyl siloxanes, polyesters, polycarbonates, and polymethyl methacrylates that are relatively difficult to form radicals. If the supporting material is a cellulosic polymer of a shape such as film, fiber, particle, hollow fiber or sponge, oxidation method with ozone or cerium ionization method is more suitable.

It is also possible to graft-polymerize the said polymeric compound in a homogeneous system, aside from the graft-polymerization in heterogeneous system, on the surface of the aforesaid supporting material.

Method of incorporating the crosslinking structure during polymerization of the monomer and a method of incorporating the crosslinking structure after completion of the polymerization process are available as the method of forming the crosslinked structure, and any one of these methods can be used in this invention.

The former method is carried out, embodically, by copolymerizing a bifunctional monomer. It can be performed by using, for example, N,N-methylenebis acrylamide, hydroxyethyl dimethyl dimethacrylate or divinyl benzene and so on.

With the latter method, it is common to form a crosslink among molecules by light, electron beam or gamma-irradiation.

The material of this invention shows an intermediary state of hydrophilicity and hydrophobicity near the temperature of its use, to suppress significantly the attachment of living body elements, particularly the blood elements. When it is used as medical material, it can suppress significantly the deterioration of medical functionality that accompanies attachment of living body elements and at the same time can reduce deposition of living body elements on the surface of the material, particularly the serious side effect caused by formation of thrombus.

Examples are illustrated below to embodically explain this invention. The range of this invention is determined by what is claimed and is not limited by the following examples.

### Example 1

N-Isopropylacrylamide monomer (NIPAAm, Eastman Kodak Co.) 50 g was dissolved in benzene 500 ml, and 2,2'-azobisisobutyronitrile (AIBN) 0.2 g was used as the polymerization initiator, to run polymerization at 60°C for 12 hours in a stream of nitrogen gas, with constant agitation. Because the polymer precipitated in benzene, it was decanted and then the precipitate was dissolved in tetrahydrofuran (THF) and then ethyl ether was used to reprecipitate and purify the polymer. LCST of the various solutions of poly-N-isopropyl acrylamide (PNIPAAm concentration: 1 w/v%) thus obtained was measured, and results are shown in Table I.

Like in water, PNIPAAm was found to show LCST in bovine serum.

**TABLE I**

| **LCST of various solutions of PNIPAAm** | |
|---|---|
| **Solution species** | **LCST (°C)** |
| **Water** | 31.8 ± 0.1 |
| **Phosphate buffer (PBS)** | 28.9 ± 0.1 |
| **Bovine serum** | 28.2 ± 0.1 |

### Example 2

NIPAAm monomer 50 g, acrylamide(AAm) monomer 3.1 g, and AIBN 0.21 g as the polymerization initiator were dissolved in THF 500 ml, and polymerization was carried out at 50°C for 12 hours in a stream of nitrogen gas, with constant agitation. After concentrating the reaction mixture two-folds by an evaporator, it was precipitated and purified with ethyl ether, to obtain a flaky polymer [Cop.(NIPAAm/AAm-1)] by drying in vacuum. Further, AAm monomer 9.3 was used against NIPAAm monomer 50 g to carry out polymerization and purification by the same method as the Cop.(NIPAAm/AAm)-1, to obtain Cop.(NIPAAm/AAm)-2. Each polymer was used to prepare a solution (polymer concentration: 5 w/v%), and their LCST was measured by turbidimetric method and shown in Table II. LCST was found to increase with the charge ratio of AAm which was a hydrophilic monomer.

**TABLE II**

| **LCST of various polymers in solutions** | | |
|---|---|---|
| **Polymer species** | **PBS** | **Bovine serum** |
| **PNIPAAm** | 28.9 ± 0.1 | 28.2 ± 0.1 |
| **Cop.(NIPAAm/AAm)-1** | 34.1 ± 0.3 | 32.6 ± 0.1 |
| **Cop.(NIPAAm/AAm)-2** | 39.0 ± 0.1 | 36.5 ± 0.3 |

### Example 3

NIPAAm monomer 50 g, n-butyl methacrylate (nBMA) monomer 3.3 g and AIBN 0.21 g as the polymerization initiator were dissolved in THF 500 ml, and polymerization was carried out at 50°C for 12 hours in a stream of nitrogen gas with constant agitation. After concentrating the reaction mixture two-fold in an evaporator, it was precipitated and purified by using ethyl ether, and thus a flaky polymer [Cop.(NIPAAm/BMA)-1] was obtained by drying in vacuum. Furthermore, n-butyl methacrylate 6.6 g was used against NIPAAm monomer 50 g to run the polymerization and purification by the same method as the case of Cop.(NIPAAm/BMA)-1 to obtain Cop.(NIPAAm/BMA)-2. Solution was prepared from each polymer (polymer concentration: 5 w/v%), and the LCST was measured by turbidimetric method and shown in Table III. It was observed that LCST declined with the increasing charging ratio of n-BMA which is a hydrophobic monomer.

**TABLE III**

| **LCST of various polymers in solutions (°C)** | | |
|---|---|---|
| **Polymer species** | **PBS** | **Bovine serum** |
| **PNIPAAm** | 28.9 ± 0.1 | 28.2 ± 0.1 |
| **Cop.(NIPAAm/BMA)-1** | 19.0 ± 0.6 | 20.5 ± 0.4 |
| **Cop.(NIPAAm/BMA)-2** | 7.8 ± 0.3 | 13.8 ± 0.2 |

### Example 4

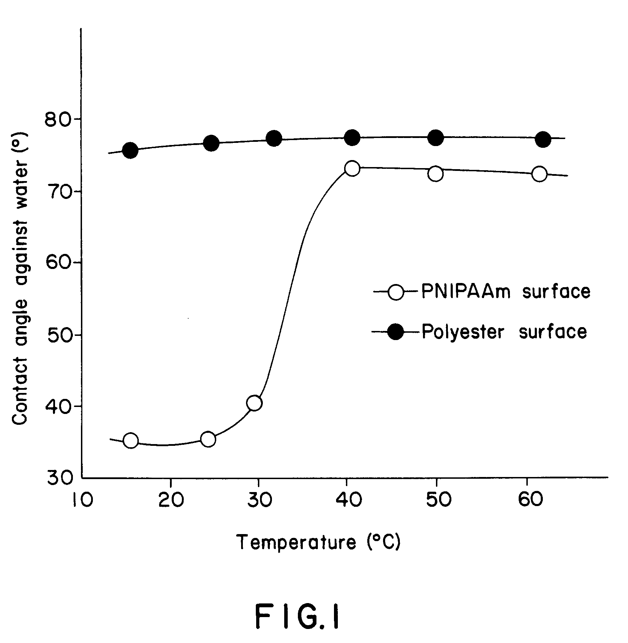

PNIPAAm synthesized in Example 1 was dissolved in THF to prepare a solution having a concentration of about 5 w/v%. Using this solution, a PNIPAAm layer having a thickness of about 10 µm was formed on a polyester film (thickness about 100 µm) by a solvent casting process. Dependency of the contact angle of water on the PNIPAAm-coated surface of said PNIPAAm coated polyester film on temperature was measured and results are illustrated in Fig. 1. As clearly demonstrated in Fig. 1, the PNIPAAm coated surface had a contact angle of less than about 40° and showed hydrophilicity when the temperature was lower than the LCST (about 32°C). But, the contact angle increased abruptly to show hydrophobicity when the temperature was higher than LCST. Thus, near the LCST, dependency of contact angle on temperature was high and it shows an intermediary state between hydrophobicity and hydrophilicity. On the other hand, with the surface of polyester film which was a substrate, the contact angle did not show a change with temperature and thus it showed hydrophobicity.

### Example 5

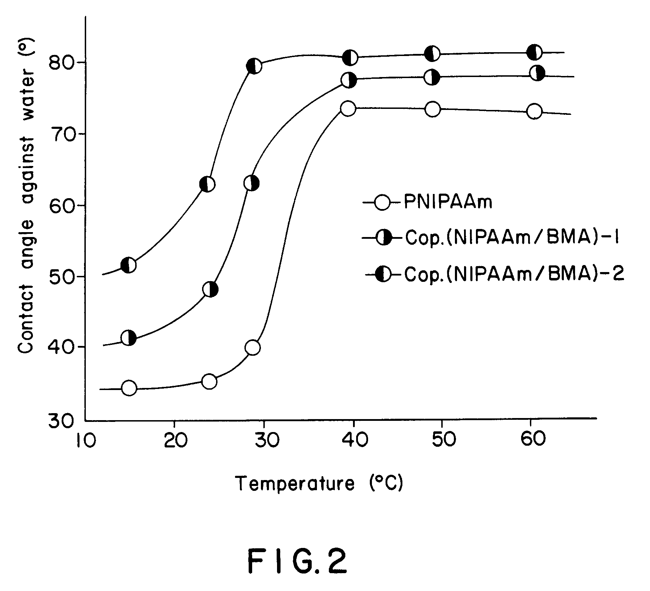

Cop.(NIPAAm/BMA)-1 and Cop.(NIPAAm/BMA)-2 synthesized in Example 3 were dissolved respectively in THF, to prepare a solution of about 5 w/v%. Using the method similar to Example 4, polymer films coated with each polymer to a thickness of about 10 µm were prepared, and dependency of the contact angle of each polymer surface against water on temperature was measured by the same method as Example 4, and shown in Fig. 2. As clearly demonstrated in Fig. 2, the temperature of the intermediary region of hydrophilicity and hydrophobicity (corresponding to LCST) of the Cop.(NIPAAm/BMA)-1 shifted to the lower temperature side, compared to PNIPAAm, meantime the contact angle in the entire temperature region was greater and therefore it was hydrophobic. This difference can be attributed to copolymerization of BMA. With the Cop.(NIPAAm/BMA)-2 which had a greater copolymerization of BMA, this trend was further intensified.

### Example 6

Experiment to attach the blood platelets of dog on the PNIPAAm surface of the PNIPAAm-coated polyester film prepared in Example 4 was carried out.

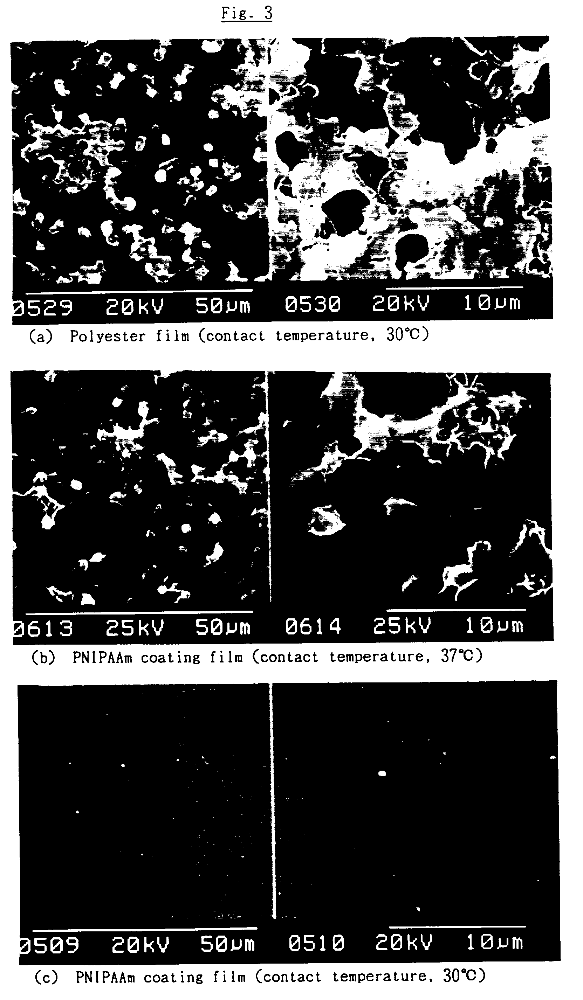

A canule was inserted into the femoral vein of adult dog to drain the blood into a plastic tube containing 1 volume of 3.8 w/v% sodium citrate solution in 9 volumes of blood. The tube was centrifuged immediately at 1000 rpm for 15 minutes, to prepare fresh platelet rich plasma. Then, PNIPAAm coated film (1 cm x 1 cm) was soaked in 2 ml of the aforesaid plasma, to establish a contact for 3 hours at various temperatures. A similar contact experiment was carried out with the polyester film that was not coated with PNIPAAm, to serve as a control experiment. After contact with the plasma, it was washed several times with 37°C phosphate buffer, and then it was immobilized with 1% glutaraldehyde-PBS, and finally washed with distilled water, and then dried in vacuum. This was used as the sample for scanning electron microscopy. The temperature used for contact with the fresh platelet rich plasma was about 37°C, about 30°C, and about 4°C. And, FT-IR (ATR) method was used to measure the peak strength at 3300-3350 cm⁻¹ and 1656-1642 cm⁻¹ which are the characteristic absorption of PNIPAAm, to confirm the remainder of the PNIPAAm-coated layer after contact with the plasma. These results are summarily shown in Table IV. And, scanning electromicrograph of typical situation of attachment of platelets is shown in Fig. 3. As clearly demonstrated in Table IV, a large amount of attachment of blood platelets was observed with the polyester film which served as a control sample, at contact temperature of 37°C, 30°C and 4°C. On the other hand, with the PNIPAAm-coated film, a large amount of attachment of blood platelets was recognized at a temperature (37°C) higher than the LCST, but no attachment of blood platelet was noted at all at 30°C which is near the LCST and at 4°C which is lower than the LCST. Thus, although the PNIPAAm did not dissolve in plasma at 30°C which is near the LCST, but it dissolved completely in plasma at 4°C. Therefore, attachment of blood platelet was not seen at all at 4°C because PNIPAAm layer dissolved at that temperature. On the other hand, at 30°C which is near the LCST, PNIPAAm layer remained completely, but no attachment of blood platelets was seen. This evidence proves that, near the LCST, the PNIPAAm layer has a property located between hydrophobic and hydrophilic properties and thus firm attachment of blood element was prevented. Thus, if the temperature of use is set at about 30°C, the said PNIPAAm-coated film will show an excellent effect of preventing the attachment of living body elements.

**Table IV**

| **Extent of attachment of blood platelet on polyester surface and PNIPAAm-coated surface, and residue of PNIPAAm on PNIPAAm-coated layer** | | | |
|---|---|---|---|
| **Sample** | **Contact temperature (°C)** | **Attachment of blood platelets** | **Residue of PNIPAAm** |
| **Polyester film** | 37 | + | - |
| | 30 | + | - |
| | 4 | + | - |
| **PNIPAAm-coated film** | 37 | + | + |
| | 30 | - | + |
| | 4 | - | - |
| + Blood platelet exists, or PNIPAAm remains. - No blood platelet, or PNIPAAm does not remain. | | | |

### Example 7

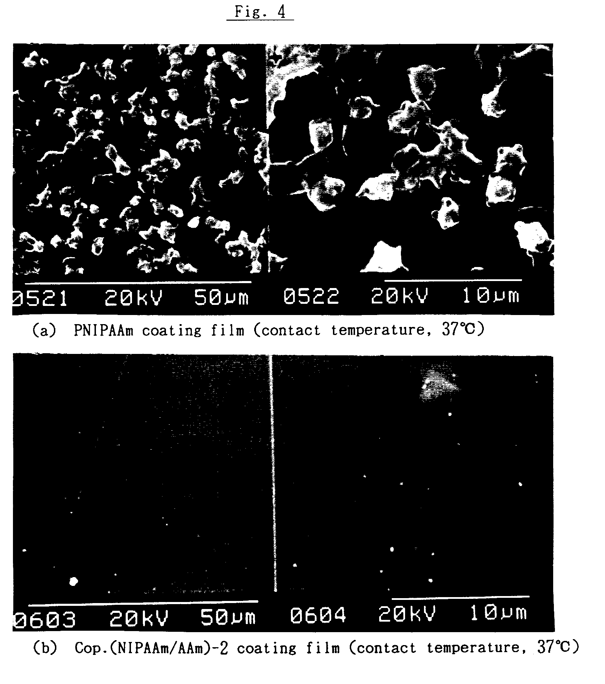

Cop(NIPAAm/AAm)-2 prepared in Example 2 was dissolved in THF to prepare a solution having a concentration of about 5 w/v%, and a polyester film coated to a thickness of about 10 µm was prepared by the same method as Example 4. The said Cop.(NIPAAm/AAm)-2 coated film and the PNIPAAm coated film used in Example 5 were contacted with a fresh platelet rich plasma by the same method as Example 5, to run a blood platelet attachment experiment. Remainder of the coated layer after contact with plasma was checked by the FT-IR method used in Example 5. With both samples, coated layer was found to remain. Scanning electron micrographs of the blood platelet attachment with both samples are illustrated in Fig. 4. As clearly demonstrated in Fig. 4, although a large amount of blood platelet attachment was noticed in the case of PNIPAAm coated film, almost no attachment of blood platelets was noticed in the case of Cop.(NIPAAm/AAm)-2 coated film. This is believed to be the closeness of the temperature (37°C) to the LCST in the case of Cop.(NIPAAm/AAm)-2. Therefore, if the temperature of use is close to the temperature of the body, i.e., 37°C, Cop.(NIPAAm/AAm)-2 can suppress effectively the attachment of blood elements.

### Example 8

The polyester film (thickness = about 100 µm, 5 cm x 5 cm) used in Example 4 was inserted into the chamber of an internal electrode type of plasma irradiation device (Samco International K.K.). After the interior of the chamber was evacuated to about 0.8 Torr, argon gas was introduced at a flow rate of 30 ml/min, and plasma irradiation was performed at an output of 50 Watt and frequency of 13.56 MHz for 15 seconds. And, immediately an aqueous 10 w/v solution of NIPAAm was introduced into the chamber to soak said polyester film in that solution, to carry out the graft polymerization at ambient temperature for 20 hours. Prior to introducing the aqueous monomer solution into the chamber, it was bubbled thoroughly with argon gas to completely remove the air dissolved and remained in the aqueous solution. A film which was plasma-irradiated without introduction of said monomer under entirely identical condition was prepared also. Contact angle of these films against water was measured at 10°C and 40°C, respectively, and shown in Table V. As clearly demonstrated in Table V, there was absolutely no difference in contact angle with untreated film, between 10°C and 40°C. With plasma-treated film, the contact angle was lower than the untreated film, but again there was no difference in contact angle between 10°C and 40°C. On the other hand, with the PNIPAAm plasma grafted film, difference of contact angle showed up clearly between 10°C and 40°C, like the case with PNIPAAm coated film. Thus, hydrophilic property was seen at 10°C, and hydrophobic property was seen at 40°C. These evidences suggest that PNIPAAm, even when grafted on the surface of the material, can undergo hydrophilic-hydrophobic transition above and below the LCST.

**Table V**

| **Contact angles of various films against water** | | |
|---|---|---|
| **Film species** | **Contact angle (°)** | |
| | **10°C** | **40°C** |
| **Polyester film without treatment** | 77 | 77 |
| **Film irradiated with plasma** | 50 | 50 |
| **PNIPAAm grafted film** | 40 | 65 |

### Example 9

Extent of attachment of blood platelets on the surface of various films prepared in Example 7 was investigated with the fresh, platelet rich plasma from dog, by the method of Example 5. Results are shown in Table VI. As clearly demonstrated in Table VI, large amount of blood platelets was found to attach on the untreated polyester film and plasma-treated polyester film, at all temperatures. On the other hand, PNIPAAm plasma grafted film, large amount of blood platelets were found to attach on the surface of the film at 37°C which is above the LCST or at 10°C which is below the LCST, but absolutely no attachment of blood plates was seen at 30°C which is near the LCST. This evidence suggests that the surface of PNIPAAm grafted film, if the temperature was set at about 30°C, can suppress effectively the attachment of blood elements in the intermediary region of hydrophobility and hydrophilicity.

**Table VI**

| **Extent of attachment of blood platelets on the surface of various films (observation by scanning electron microscopy)** | | |
|---|---|---|
| **Sample** | **Contact temperature (°C)** | **Attachment of platelets** |
| **Polyester film without treatment** | 37 | + |
| | 30 | + |
| | 10 | + |
| **Polyester film treated with plasma** | 37 | + |
| | 30 | + |
| | 10 | + |
| **PNIPAAM plasma grafted film** | 37 | + |
| | 30 | - |
| | 10 | - ∼ + |
| + : Attachment of blood platelets - : No attachment of blood platelets | | |

### Example 10

NIPAAm 10 g and water 10 ml were added to a conical flask, and then a 0.1N aqueous nitrate solution prepared by dissolving cerium (II) ammonium nitrate 27 mg in water 10 ml was added dropwise to the flask that contained a cellulose acetate (CA) film (Millipore Co., fractionated molecular weight = 1000, diameter = 30 mm, thickness of the film = 100 µ), to carry out graft reaction at 35°C for 2 hours in a stream of nitrogen gas. After the reaction, the film was washed with water and then dried in vacuum, to obtain PNIPAAm-g-CA film. Water content of this PNIPAAm-g-CA film was about 18% at 37°C. In contrast, the water content was about 55% at 10°C. Thus, it clearly showed the hydrophilicity-hydrophobicity change of PNIPAAm.

## Claims

1. A method for substantially preventing adhesion of a living body element to a material comprising
(a) forming said material comprising a temperature responsive polymeric compound;
(b) maintaining said material at a temperature near the LCST of said temperature responsive polymer; and
(c) contacting a fluid comprising said living body element with said polymer adhered material.

2. The method of Claim 1 wherein the temperature responsive polymer is selected from the group consisting of a poly-N-substituted acrylamide derivative, a poly-N-substituted methacrylamide derivative or its copolymer, polyvinvylmethyl ether or a partially acetylated polyvinyl alcohol.

3. The method of Claim 1 wherein the maintenance temperature is within 5°C of the LCST of said temperature responsive polymer.

4. The method of Claim 1 wherein step (a) comprises adhering said temperature responsive polymer to a support.

5. The method of Claim 4 wherein said temperature responsive polymer is coated on said support.

6. The method of Claim 4 wherein said temperature responsive polymer is graft polymerized to said support.

7. The method of Claim 1 wherein said temperature responsive polymer has a crosslinked structure.

8. The method of Claim 1 wherein said fluid comprises blood.

9. The method of Claim 1 which prevents thrombus formation.

10. A biocompatible material which is substantially nonadhesive to living body elements comprising a temperature responsive polymeric compound at a temperature near the LCST of said temperature-responsive polymer.

11. The biocompatible material of Claim 10 wherein said temperature responsive polymer is in partial adherence with a support.

12. The biocompatible material of Claim 10 wherein the temperature responsive polymer is selected from the group consisting of a poly-N-substituted acrylamide derivative, a poly-N-substituted methacrylamide derivative or its copolymer, polyvinvylmethyl ether or a partially acetylated polyvinyl alcohol.

13. The biocompatible material of Claim 10 wherein the temperature is within 5°C of the LCST of said temperature responsive polymer.

14. The biocompatible material of Claim 11 wherein said temperature responsive polymer is coated on said support.

15. The biocompatible material of Claim 11 wherein said temperature responsive polymer is graft polymerized to said support.

16. The biocompatible material of Claim 10 wherein said temperature responsive polymer has a crosslinked structure.
